Europäisches Patentamt

European Patent Office

Office européen des brevets

(11) Veröffentlichungsnummer: **0 362 466**
**A2**

(12) # EUROPÄISCHE PATENTANMELDUNG

(21) Anmeldenummer: **89106487.5**

(22) Anmeldetag: **12.04.89**

(51) Int. Cl.⁵: **A61B 17/00**

(30) Priorität: **06.10.88 DE 3833993**

(43) Veröffentlichungstag der Anmeldung:
**11.04.90 Patentblatt 90/15**

(84) Benannte Vertragsstaaten:
**AT BE CH DE ES FR GB GR IT LI LU NL SE**

(71) Anmelder: **Messerschmitt-Bölkow-Blohm**
**Gesellschaft mit beschränkter Haftung**
**Robert-Koch-Strasse**
**D-8012 Ottobrunn(DE)**

(72) Erfinder: **Hessel, Stefan, Dr.**
**Evastrasse 34**
**D-8000 München 81(DE)**
Erfinder: **Frank, Frank, Dr.**
**Sarreiterweg 13**
**D-8017 Ebersberg(DE)**
Erfinder: **Wondrazek, Fritz, Dr.**
**Riegelstrasse 27**
**D-8068 Pfaffenhofen(DE)**

(54) **Lichtleiter- und Bestrahlungseinrichtung.**

(57) Zur Laserlichtbestrahlung von insbesondere biologischen Materialien mittels eines Lichtleiters (2) zur Laserlichtübertragung von der Lichtquelle zur Applikationsstelle werden mehrere voneinander unabhängige Strahlenbündel (14, 16) durch einen einzigen gemeinsamen Lichtleiter in der Weise übertragen, daß dem Lichtleiter auf der Eintrittsseite (6) zwischen Lichtquelle und proximalem Leiterende mehrere unterschiedliche Einkoppelwinkelbereiche (10, 12) und auf der Austrittsseite (8) zwischen distalem Leiterende und Applikationsstelle mehrere unterschiedliche, jeweils einem der Einkoppelwinkelbereiche entsprechende Auskoppelwinkelbereiche (18, 20) zugeordnet sind und jeder Ein- und zugeordnete Auskoppelwinkelbereich (10, 18 bzw. 12, 20) von jeweils einem der voneinander unabhängigen Strahlenbündel (14 bzw. 16) durchsetzt ist.

Fig.1

## LICHTLEITER- und BESTRAHLUNGSEINRICHTUNG

Die Erfindung bezieht sich auf eine Lichtleitereinrichtung, und insbesondere ein medizinisches Applikationsteil, nach dem Oberbegriff des Patentanspruchs 1, sowie eine Bestrahlungseinrichtung für insbesondere biologische Materialien mittels optischer Strahlung, nach dem Oberbegriff des Patentspruchs 2.

Es ist bekannt, daß Laserstrahlen bei medizinischer, aber auch anderer, technischer Anwendung je nach Wellenlänge, Betriebsart (cw-, Puls- oder Q-switch-Betrieb) und Bestrahlungsparametern, wie Bestrahlungsenergie und -intensität, oder Abstrahlcharakteristik, an der Applikationsstelle unterschiedliche Wirkungen auslösen. So hat etwa die 1,06 μm-Strahlung eines Neodym-YAG-Lasers auf biologische Gewebe einen mehr koagulierenden Effekt, während eine langwelligere Laserstrahlung eine mehr schneidene Wirkung besitzt. Sollen die verschiedenartigen Bestrahlungswirkungen nacheinander oder gleichzeitig jeweils dosiert in unterschiedlichen Teilzonen der Applikationsstelle erreicht werden, so ergibt sich die Schwierigkeit, daß bei den bekannten Einrichtungen der beanspruchten Art wegen der beengten Platzverhältnisse, z. B. in Lumen von organischen Hohlgefäßen, lediglich ein einziger Lichtleiter ohne Umschalt- oder Steuerungsmöglichkeit am distalen Ende zur Verfügung steht und daher eine solche multifunktionale Anwendung nur in sehr begrenztem Umfang und auch dann nur durch einen nicht immer zulässigen und jedenfalls bedienungsaufwendigen Austausch des Applikationssystems einschließlich des Lichtleiters möglich ist.

Aufgabe der Erfindung ist es, eine Einrichtung der beanspruchten Art so auszubilden, daß mit einem einzigen Lichtleiter auf baulich einfache Weise und ohne Steueraufwand am distalen Ende mehrere verschiedenartige, jeweils individuell dosierbare Bestrahlungswirkungen in räumlich voneinander getrennten Teilzonen der Applikationsstelle zu erzielen sind.

Diese Aufgabe wird erfindungsgemäß durch die im Anspruch 1 gekennzeichnete Lichtleitereinrichtung bzw. die im Anspruch 2 gekennzeichnete Bestrahlungseinrichtung gelöst.

Erfindungsgemäß werden mehrere, voneinander unabhängige Strahlenbündel von einer einzigen Lichtleitfaser in der Weise übertragen, daß sie unter jeweils unterschiedlichen Einkoppelwinkeln in das proximale Lichtleiterende eingestrahlt werden und aufgrund dadurch ausgenutzter, spezifischer Eigenschaften des Lichtleiters in räumlich voneinander abgegrenzten Auskoppelwinkelbereichen am distalen Leiterende austreten, ohne daß es hier irgendwelcher Trenn- oder gar Steuerorgane für die emittierten Strahlenbündel bedarf. Mit den erfindungsgemäßen Einrichtungen gelingt es allein durch proximalseitige Steuereingriffe, für die - anders als am distalen Leiterende - in baulicher Hinsicht ausreichend Raum vorhanden ist, mehrere unterschiedliche Applikationswirkungen jeweils einzeln dosierbar wahlweise simultan oder wechselweise in verschiedenen, sich gewünschtenfalls aber auch überdeckenden Teilzonen der Applikationsstelle zu erzielen. Die Erfindung eignet sich daher in hervorragender Weise für medizinische Anwendungsfälle in beengten Behandlungsfeldern, wo der therapeutische Gesamterfolg von der Synergiewirkung mehrerer verschiedenartiger und jeweils einzeln dosier barer optischer Bestrahlungs- und insbesondere Laserlichteffekte abhängt.

In weiterer vorteilhafter Ausgestaltung der Erfindung sind die Ein- und Auskoppelwinkelbereiche gemäß Anspruch 3 vorzugsweise bezüglich der Lichtleiter-Mittelachse radialsymmetrisch gestaffelt, so daß sich auch bei Lichtleitern mit begrenzter maximaler Apertur noch problemlos eine deutliche Trennung der einzelnen Strahlenbündel erzielen läßt. Aus dem gleichen Grund werden diese gemäß Anspruch 4 zweckmäßigerweise jeweils in Form konvergenter, auf die Lichtleiter-Eintrittsfläche fokussierter Lichtkegel eingekoppelt. Für die getrennte Lichtbündel-Übertragung ist bei Laserlicht-Wellenlängen in der Grössenordnung von 3 μm is 2 μm als Lichtleiter, wie gemäß Anspruch 5 bevorzugt, eine übliche Multimode-Lichtleiter-Glasfaser vollständig ausreichend.

Die unterschiedlichen Einzeleffekte der Strahlenbündel werden gemäß Anspruch 6 vorzugsweise dadurch erzielt, daß sich die Strahlenbündel hinsichtlich Strahlungsleistung, Betriebsart und/oder Wellenlänge voneinander unterscheiden bzw. unabhängig voneinander steuerbar sind. In baulich besonders einfacher Weise werden die Strahlenbündel gemäß Anspruch 7 zweckmäßigerweise nach Art einer Zweipunkt-Steuerung selektiv in das proximale Lichtleiterende eingekoppelt.

Für die verschiedenartigen Lichtbündel ist gemäß Anspruch 8 eine mehrteilige Lichtquelle mit mehreren, voneinander getrennten Lichterzeugern vorgesehen. Gemäß einer zweiten, baulich wesentlich einfacheren Variante wird jedoch gemäß Anspruch 9 eine einteilige Lichtquelle mit einem nachgeschalteten Strahlungsteiler zur Aufspaltung der Lichtstrahlung in die einzelnen Strahlenbündel verwendet, wobei in diesem Fall für Strahlenbün del unterschiedlicher Wellenlänge gemäß Anspruch 10 bevorzugt ein eine multichromatische Lichtstrahlung emittierender Laser und als Strahlteiler ein die Laserstrahlung wellenlängenabhängig aufspaltendes Prisma vorgesehen ist. In baulich besonders einfacher Weise wird die selektive Ansteuerung der einzelnen Einkoppelwinkelbereiche mit den zugeordneten Strahlenbündeln gemäß Anspruch 11 zweckmäßigerweise durch einen umschaltbaren Spiegel- oder ein ähnliches lichtablenkendes oder -sperrendes Schaltelement mit mindestens einer aktiven, jeweils einem der Einkoppelwinkelbereiche zugeordneten Schaltposition bewirkt.

Anhand der in den Figuren schematisch dargestellten Ausführungsbeispiele wird die Erfindung nachfolgend näher beschrieben. Es zeigen:

Fig. 1 einen Lichtleiter zur Übertragung zweier voneinander unabhängiger Strahlenbündel einschließlich der jeweils zugeordneten, unterschiedlichen Ein- und Auskoppelwinkelbereiche;

Fig. 2 eine Bestrahlungseinrichtung mit einem am proximalseitigen Lichtleiterende angeordneten, selektiv verstellbaren Lichtdeflektor in Form eines Prismas zum wechselweisen Umschalten der von einer Lichtquelle emittierten Strahlung auf unterschiedliche Auskoppelwinkelbereiche;

Fig. 3 eine Bestrahlungseinrichtung mit einem zwischen Lichtquelle und Lichtleiter angeordneten Strahlteiler zur Aufspaltung der Lichtstrahlung in zwei parallele, jeweils unter unterschiedlichen Winkeln ein- und ausgekoppelte Lichtbündel;

Fig. 4 eine Bestrahlungeinrichtung mit einer einzigen Laserlicht-Quelle für gleichzeitig zwei Wellenlängen einschließlich des ein- und auskoppelseitigen Strahlenverlaufs; und

Fig. 5 eine Bestrahlungseinrichtung mit zweiteiliger Lichtquelle zur getrennten Lichtbündelerzeugung einschließlich der jeweiligen Ein- und Abstrahlcharakteristika.

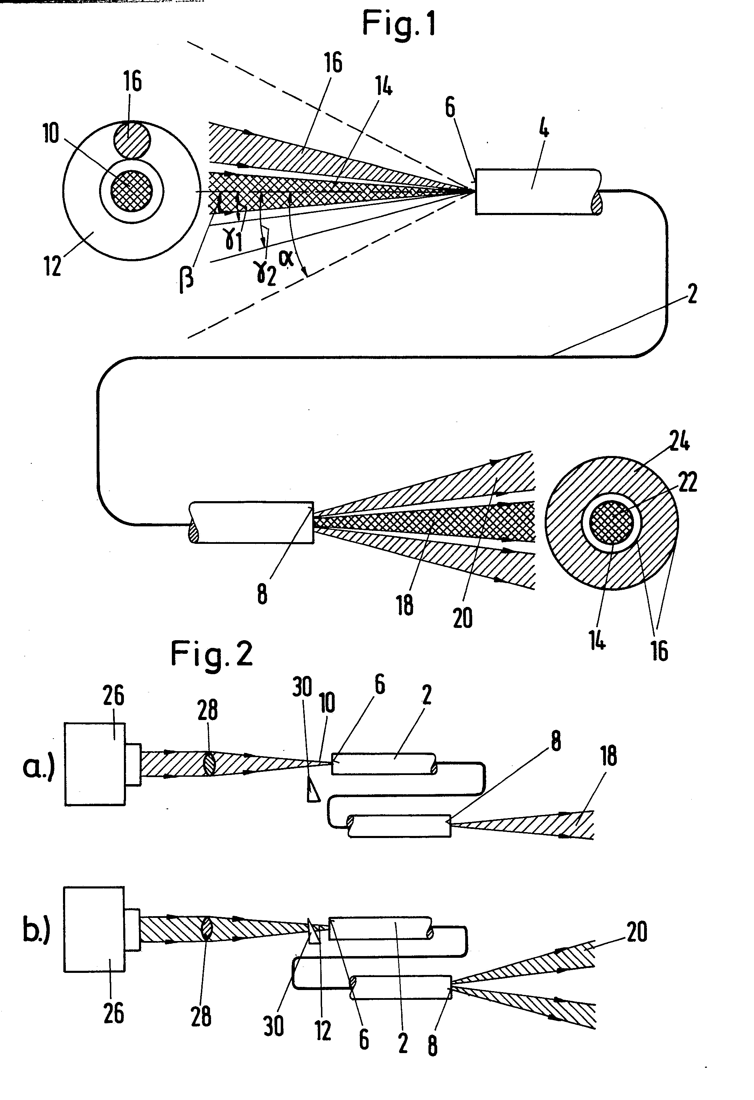

Fig. 1 zeigt einen Lichtleiter 2 in Form einer Multimode-Einzelfaser mit senkrecht zur Lichtleiterachse 4 verlaufenden Ein- und Austrittsflächen 6, 8 und einem durch den Öffnungswinkel $\alpha$ definierten Akzeptanzkegel. Dem Lichtleiter 2 sind am proximalen Leiterende innerhalb des Akzeptanzkegels zwei unterschiedliche Einkoppelwinkelbereiche zugeordnet, nämlich ein innerer Einkoppelwinkelbereich 10, der durch einen auf die Eintrittsfläche 6 gerichteten, zur Leiterachse 4 koaxialen Zentralkegel mit dem halben Scheitelwinkel $\beta$ gebildet wird, sowie ein den inneren Kegelbereich 10 ringförmig umschließender, äußerer Einkoppelwinkelbereich 12, welcher von ebenfalls auf die Eintrittsfläche 6 gerichteten und zur Lichtleiterachse 4 koaxialen Kegelmantelflächen mit jeweils gegenüber dem Winkel $\beta$ stufenweise erhöhten, halben Scheitelwinkeln $\gamma_1$ bzw. $\gamma_2$ begrenzt wird.

Innerhalb jedes Einkoppelwinkelbereichs 10, 12 wird jeweils ein konvergentes Strahlenbündel, nämlich das kreuzschraffierte, achsensymmetrische Strahlenbündel 14 einerseits und das schräg eingestrahlte Strahlen bündel 16 andererseits auf die Eintrittsfläche 6 des Lichtleiters 4 fokussiert, und aufgrund der spezifischen Transmissionseigenschaften einer Lichtleitfaser 2 wird das zentrale Strahlenbündel 14 am distalen Leiterende in einem dem Einkoppelwinkelbereich 10 entsprechenden, achsensymmetrischen Auskoppelwinkelbereich 18 abgestrahlt, während das schräge Strahlenbündel 16 den Lichtleiter 2 innerhalb eines dem Einkoppelwinkelbereich 16 entsprechenden Auskoppelwinkelbereiches 20 als vom zentralen Strahlenbündel getrenntes, ringförmiges Lichtbündel verläßt. Die Größe der Einkoppelwinkelbereiche 10, 12 ist unter Berücksichtigung der numerischen Apertur des Lichtleiters 2 dabei so zu wählen, daß bei möglichst geringen Transmissionsverlusten sich die Strahlenbündel 14, 16 an der Auskoppelseite nicht überlappen. Auf diese Weise lassen sich allein durch Steuereingriffe von der Einkoppelseite des Lichtleiters 2 her gleichzeitig oder zeitlich hintereinander mehrere, voneinander unabhängige Strahlenbündel 14, 16 am distalen Leiterende unter verschiedenen Abstrahlcharakteristika und gewünschtenfalls verschiedenen Wellenlängen in voneinander getrennten Teilzonen 22, 24 der Applikationsstelle zur Einwirkung bringen, ohne den Lichtleiter 2 auswechseln zu müssen.

Die Bestrahlungseinrichtung gemäß Fig. 2a, b enthält neben dem in Fig. 1 gezeigten Lichtleiter 2 eine Laserlichtquelle 26 und eine zwischen dieser und dem proximalen Leiterende wirksamen Optik mit einer Fokussierlinse 28 und einem unmittelbar vor der einkoppelseitigen Endfläche 6 des Lichtleiters 2 angeordneten, selektiv in den Strahlengang der Fokussierlinse 28 einschwenkbaren Lichtdeflektor 30 in Form eines Prismas, durch das das konvergente Laserlichtbündel wechselweise zwischen dem achsensymmetrischen, zentralen Einkoppelwinkelbereich 10 (Fig. 2a) und dem hierzu schräg ver setzten Einkoppelwinkelbereich 12 umschaltbar ist, wodurch die Abstrahlcharakteristik am distalen Leiterende 8 derart verändert wird, daß die Laserlichtstrahlung dort einmal in einem dem Einkoppelwinkelbereich 10 entsprechenden, zentralen Auskoppelwinkelbereich 18 und dann in der aktiven Schaltposition des Prismas 30 in den ringkegelförmigen Auskoppelwinkelbereich 20 abgestrahlt wird.

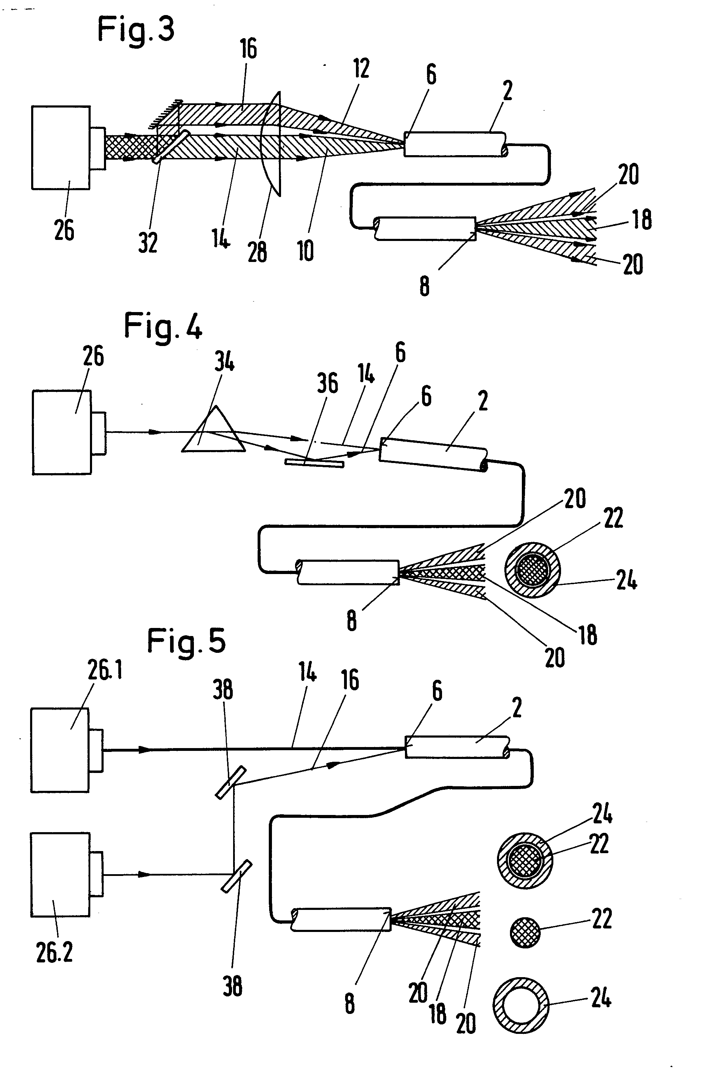

Bei der Bestrahlungseinrichtung gemäß Fig. 3 wird durch einen parallelen Versatz eines von einer Laserlichtquelle 26 erzeugten Laserstrahls vor der einkoppelseitigen Fokussierlinse 28 eine selektiv steuerbare Abstrahlcharakteristik am distalseitigen Ende 8 des Lichtleiters 2 bewirkt, wobei gemäß Fig. 3 ein Strahlteiler 32 vor der Fokussierlinse 28 angeordnet ist, der den Laserstrahl in zwei parallele Bündel 14, 16 aufteilt, so daß sich am distalseitigen Leiterende 8 gleichzeitig beide Abstrahlcharakteristika 18 und 20 ergeben. Wird anstelle des Strahlteilers 32 ein selektiv einschwenkbarer Spiegel verwendet, so läßt sich - ähnlich wie bei der Einrichtung gemäß Fig. 2 - eine wechselweise Umschaltung des Laserstrahls zwischen dem zentralen und dem ringkegelförmigen Auskoppelbereich 18 und 20 erreichen.

Fig. 4 zeigt eine Bestrahlungseinrichtung mit einer Laserlichtquelle 26, die gleichzeitig zwei unterschiedliche Wellenlängen, z. B. eine 1,06 μm- und eine 1,32 μm-Strahlung erzeugt. Die Laserstrahlung wird zunächst durch ein Prisma 34 hinsichtlich der Wellenlänge getrennt, und anschließend wird die 1,32 μm-Strahlung 14 axial, also innerhalb des zentralen Einkoppelwinkelbereichs 10 gemäß Fig. 1, und die 1,06 μm-Strahlung 16 mit Hilfe eines Reflektors 36 schräg, also innerhalb des Einkoppelwinkelbereichs 12, auf das proximale Leiterende 6 fokussiert, so daß die langwelligere Strahlung am distalen Leiterende 8 innerhalb des Auskoppelwinkelbereichs 18 austritt und auf die zentrale Teilzone 22 der Applikationsstelle auftrifft, während die kurzwelligere 1,06 μm-Strahlung vom distalen Leiterende 8 mit der ringkegelförmigen Abstrahlcharakteristik 20 emittiert wird und an der Applikationsstelle in der Ringzone 24 zur Einwirkung gebracht werden kann.

Die Bestrahlungseinrichtung gemäß Fig. 5 enthält eine zweiteilige Lichtquelle mit zwei unabhängig voneinander steuerbaren Lasern zur Erzeugung der z. B. hinsichtlich Wellenlänge, Betriebsart und /oder Leistung verschiedenartigen Laserlichtbündel 14, 16, von denen das eine - wiederum entsprechend dem Einkoppelwinkelbereich 10 gemäß Fig. 1 - axial und das andere mit Hilfe eines Spiegelsystems 38 innerhalb des Einkoppelwinkelbereichs 12 gemäß Fig. 1 schräg in das proximale Leiterende 6 eingekoppelt wird. Je nachdem, ob beide Strahlenbündel 14, 16 gleichzeitig oder zeitversetzt eingestrahlt werden, lassen sich am distalen Leiterende 8 entweder beide Abstrahlcharakteristika 18, 20 simultan erreichen oder es kann abwechselnd von der einen auf die andere Abstrahlcharakteristik umgeschaltet werden.

Die beschriebenen Lichtleiter- und Bestrahlungsanordnungen eignen sich insbesondere zur medizinischen Laserapplikation mit dünnen Endoskopen oder Kathetern. So erzeugen z. B. in der Gastroenterologie und Pneumologie thermisch wirkende Neodym-YAG-Laser mit einem herkömmlichen Lichtleiter bei senkrechtem Auftreffen der Laserstrahlung auf das Gewebe eine kreisförmige bestrahlte Fläche mit einem ausgeprägten Temperaturmaximum im Flächenmittelpunkt. Mit den erfindungsgemäßen Einrichtungen hingegen läßt sich durch gezielte Dosierung der beiden Strahlenbündel 14, 16, also etwa durch eine einfache Umschaltung der Einkoppelung entsprechend den Fig. 2, 3 oder 5, eine unabhängig von der zentralen Teilzone 22 bestrahlte Ringfläche 24 erzeugen und dadurch eine wesentlich gleichmäßigere Temperaturverteilung mit einem vorzugsweise koagulierenden Effekt auf das biologische Gewebe erzielen. Wird dagegen eine blutarme abtragende oder schneidene Wirkung des Laserstrahls gewünscht, so lassen sich beide Abstrahlcharakteristika simultan verwenden. So wird etwa mit den Einrichtungen gemäß den Fig. 4 und 5 das Gewebe durch die langwelligere Laserstrahlung im Zentrum der Applikationsstelle vaporisiert, während die umliegende Ringzone 24 durch die kurzwelligere Laserstrahlung koaguliert wird.

## Ansprüche

1. Lichtleitereinrichtung, insbesondere medizinisches Applikationsteil, zur Lichtwellen-, insbesondere Laserlichtübertragung von einer Lichtquelle zu einer Applikationsstelle,
dadurch **gekennzeichnet, daß**
zur Übertragung mehrerer voneinander unabhängiger optischer Strahlenbündel (14, 16) ein einziger gemeinsamer Lichtleiter (2) vorgesehen und diesem auf der Eintrittsseite zwischen Lichtquelle und proximalem Leiterende (6) mehrere unterschiedliche Einkoppelwinkelbereiche (10, 12) und auf der Austrittsseite zwischen distalem Leiterende (8) und Applikationsstelle (22, 24) mehrere unterschiedliche, jeweils einem der Einkoppelwinkelbereiche entsprechende Auskoppelwinkelbereiche (18, 20) zugeordnet sind und jeder Ein- und zugeordnete Auskoppelwinkelbereich (10, 18 bzw. 12, 20) von jeweils einem der voneinander unabhängigen Strahlenbündel (14 bzw. 16) durchsetzt ist.

2. Bestrahlungseinrichtung für insbesondere biologische Materialien mittels optischer Strahlung, mit einem Lichtleiter zur Lichtwellen-, insbesondere Laserlichtübertragung von einer Lichtquelle zu einer Applikationsstelle, sowie mit einer zwischen Lichtquelle und proximalem Leiterende angeordneten Optik zur Einkoppelung der von der Lichtquelle erzeugten Strahlung in den Lichtleiter,
dadurch **gekennzeichnet, daß**
mehrere, voneinander unabhängige Strahlbenbündel (14, 16) durch die Optik (28 bis 38) in jeweils unterschiedlichen Einkoppelwinkelbereichen (10, 12) in die Eintrittsfläche (6) eines einzelnen Lichtleiters (2) eingestrahlt werden derart, daß sie am distalen Leiterende (8) getrennt voneinander in jeweils unterschiedlichen Auskoppelwinkelbereichen (18, 20) austreten.

3. Einrichtung nach Anspruch 1 oder 2,
dadurch **gekennzeichnet, daß**
der eine Ein- und der zugordnete Auskoppelwinkelbereich (10, 18) durch einen zur Lichtleiterachse (4) zentrischen Innenkegel gebildet ist, der ringförmig von mehreren, zum Innenkegel koaxialen, jeweils einen weiteren Ein- bzw. zugeordneten Auskoppel-winkelbereich (12, 20) begrenzenden Kegelmantel-flächen mit jeweils stufenweise bis zum Akzeptan-zwinkel ($\alpha$) des Lichtleiters (2) erhöhtem Öffnungs-winkel ($\gamma_1, \gamma_2$) umschlossen ist. 1

4. Einrichtung nach Anspruch 3,
dadurch **gekennzeichnet,** daß
die Strahlenbündel (14, 16) in den Einkoppelwinkelbereichen (10, 12) jeweils als konvergente, auf die Lichtleiter-Eintrittsfläche (6) fokussierte Lichtkegel ausgebildet sind.

5. Einrichtung nach einem der vorhergehenden Ansprüche,
dadurch **gekennzeichnet,** daß
der Lichtleiter (2) eine Multimode-Lichtleitfaser ist.

6. Einrichtung nach einem der vorhergehenden Ansprüche,
dadurch **gekennzeichnet,** daß
die Strahlenbündel (14, 16) hinsichtlich Strahlungsleistung, Betriebsart und/oder Wellenlänge ver-schiedenartig bzw. unabhängig voneinander steuer-bar sind.

7. Einrichtung nach einem der vorhergehenden Ansprüche,
dadurch **gekennzeichnet,** daß
die Strahlenbündel (14, 16) selektiv in das proximale Leiterende einkoppelbar sind.

8. Einrichtung nach einem der vorhergehenden Ansprüche,
dadurch **gekennzeichnet,** daß
eine mehrteilige Lichtquelle (26.1, 26.2) zur getrennten Erzeugung mindestens zweier, unter unterschiedlichen Einkoppelwinkeln auf den Lichtleiter (2) gerichteter Strahlenbündel (14, 16) vorgesehen ist.

9. Einrichtung nach einem der Ansprüche 1 - 7,
dadurch **gekennzeichnet,** daß
eine einzige Lichtquelle (26) vorgesehen ist und die Optik einen Strahlteiler (32, 34) zur Aufteilung der Lichtstrahlung auf die einzelnen Einkoppelwinkelbereiche (10, 12) enthält.

10. Einrichtung nach Anspruch 9,
dadurch **gekennzeichnet,** daß
die Lichtquelle (26) als Laser mit gleichzeitig mehreren Wellenlängen ausgebildet und als Strahlteiler ein Prisma (34) zur Aufteilung des Laserlichtes in jeweils monochromatische Lichtbündel (14, 16) vorgesehen ist.

11. Einrichtung nach einem der vorhergehenden Ansprüche,
dadurch **gekennzeichnet,** daß
die Optik mindestens einen umschaltbaren Lichtdeflektor (30, 32, 36, 38) mit einer oder mehreren aktiven, jeweils einem der Einkoppelwinkelbereiche (12) zugeordneten Schaltpositionen enthält.

10565

Fig.1

Fig.2

a.)

b.)

10565

# Fig. 3

# Fig. 4

# Fig. 5